# EUROPEAN PATENT SPECIFICATION

(11) **EP 1 202 060 B1**
(45) Date of publication and mention of the grant of the patent: **13.07.2005**
(21) Application number: 01934390.4
(22) Date of filing: 29.05.2001
(51) Int. Cl.: G01N 33/543

(54) **BIOSENSOR AND METHOD FOR ITS PREPARATION**
BIOSENSOR UND VERFAHREN ZU DESSEN HERSTELLUNG
BIOCAPTEUR ET PROCEDE POUR SA FABRICATION

(30) Priority: 29.05.2000 JP 2000158759
(43) Date of publication of application: 02.05.2002
(73) Proprietor: MATSUSHITA ELECTRIC INDUSTRIAL CO., LTD., Kadoma-shi, Osaka 571-8501 (JP)
(72) Inventor: NADAOKA, Masataka, Iyo-shi, Ehime 799-3113 (JP); TAKAHASHI, Mie, Niihama-shi, Ehime 792-0026 (JP); TANAKA, Hirotaka, Matsuyama-shi, Ehime 791-1102 (JP); KITAWAKI, Fumihisa, Kadoma-shi Osaka 571-0064 (JP)
(74) Representative: Leeming, John Gerard
(86) International application number: PCT/JP2001/004498
(87) International publication number: WO 2001/092884

(56) References cited:
- EP-A- 0 903 584
- JP-A- 6 167 497
- JP-A- 7 092 156
- JP-A- 11 044 689
- JP-A- 11 094 817
- JP-A- 62 022 063
- JP-A- 2000 055 919
- US-A- 4 756 828
- PATENT ABSTRACTS OF JAPAN vol. 010, no. 048 (P-431), 25 February 1986 (1986-02-25) & JP 60 192249 A (MATSUSHITA DENKO KK), 30 September 1985 (1985-09-30)

## Description

### TECHNICAL FIELD

The present invention relates to a biosensor and, more particularly, to a biosensor utilizing a chromatography and a manufacturing method thereof.

### BACKGROUND ART

A conventional biosensor is provided with a development layer for developing an inspection target solution, which includes a reagent immobilization part where a reagent is immobilized and a reagent holding part where a marked reagent is held in a dry state to be eluted by development of the inspection target solution in parts thereof, and measures a bonding amount of the marked reagent in the reagent immobilization part, thereby qualitatively or quantitatively measuring components to be measured in the inspection target solution. An example of such biosensor is an immuno-chromatographic sensor.

The immuno-chromatographic sensor is generally constituted by an application layer where an inspection target solution is applied, plural development layers, and a water absorbing layer provided at the end of the immuno-chromatographic sensor, and an antibody immobilization part where an antibody for a measurement target in the inspection target solution is immobilized is held in a part of the development layer. Further, an antibody which is marked on the application layer side with respect to the antibody immobilization part and for an epitope different from that for the antibody immobilized in the antibody immobilization part is held in a marked reagent holding part, in a dry state from which the antibody can be eluted by the inspection target solution. A reaction mode of such chromatographic sensor is referred to as a sandwich reaction, in which a sandwich complex of the immobilized antibody, the measurement target in the inspection target solution, and the marked antibody is formed. When the inspection target solution is a dimeric or more antigen, for example, that has the identical structure in the identical molecule, the antibody for an epitope different from that for the immobilized antibody need not be held but an antibody for the same epitope as that for the immobilized antibody may be held in the marked reagent holding part.

US 5,451,350 discloses an analytical device that determines the analytes in a liquid sample. This device includes a layer of flat material which has a sample application area and a detection area thereupon. The detection area includes a reagent for determining the analyte, and the absorbent sample application area and the absorbent detection area are disposed within a liquid impermeable boundary. The liquid permeable boundary may be formed by a suitable heat treatment, such as laser irradiation.

US 4,756,828 discloses a device for use in a chromatographic system wherein a component of a mixture is partitioned between a liquid phase and an immobile phase. The device comprises at least one strip of a bibulous material. The strips are prepared from a sheet of a bibulous material by non-deformative or non-compressive cutting of the sheet. The preferred cutting means is a laser beam.

The English language abstract to JP 60192249 discloses the acquisition of an extract blood sugar value by destroying preliminarily the blood components in a sample by ultrasonic wave bringing the grape sugar in the sample into reaction with a biosensor prior to bringing the sample into contact with the biosensor.

Next, a measuring method of this immuno-chromatographic sensor will be described.

First, a required amount of inspection target solution is applied to the application layer and the inspection target solution permeates the development layer, so as to start the measurement. Then, a measurement result is obtained by the marked antibody bonded to the immobilized antibody in the antibody immobilization part. A typical marker of this marked antibody is gold colloid particles or the like, and the antibody is marked with the gold colloid particles or the like, so that bonding of the measurement target and the antibody in the antibody immobilization part can be seen by the gold colloid particles, thereby a visual measurement result can be obtained.

While a sandwich reaction of an antigen antibody reaction is adopted as a measurement principle, other competitive reactions can be also adopted as measurement principles. Also in these cases, a bonding state of the marked antibody in the antibody immobilization part (or an antigen immobilization part when the measurement target is an antibody) is confirmed, so as to obtain the measurement result.

Further, while a description is given of a case where a visual qualitative judgement is required as a method for obtaining a measurement result, in a case where a semi-quantitative or more accurate judgement is required as a method for obtaining a measurement result, such methods are also available as a method for reading a reflective absorbance employing a densito-pattern analyzer as a typical reflective spectrophotometer, a method for performing reading with a transparent mode employing an optical reading device, which is disclosed in Japanese Published Patent Application No. Hei.10-274624, and a method for taking-in a measurement result as an image by a camera or the like to be processed arithmetically, which is disclosed in Japanese Published Patent Application No. Hei.10-274653.

This immuno-chromatographic sensor is widely utilized as one which meets the demand of a quick, simple, accurate, low-cost, and easily available measuring device, which comes from a concept of POC (Point of Care) in the medical and diagnostic scene in recent years, for a diagnosis in restricted measurement items not only in the medical scene but also in household.

However, in a measurement employing the immuno-chromatographic sensor, an artificial manipulation is difficult in the process of the inspection target solution permeating, which results in dependence on the permeability of the development layer. This achieves simplification in the operationlity, which is an advantage of the immuno-chromatographic sensor, but results in defects in accuracy at the same time. That is, in the immuno-chromatographic sensor in which parallel side faces of the development layer are opened, a permeation rate of the inspection target solution is not uniform, and thus development of the measurement target as well as development of the marked reagent are not uniform under this condition, whereby it is difficult to obtain high accuracy in a quantitative measurement. Further, the so-constituted immuno-chromatographic sensor can only have semi-quantitative performance with low qualitative or quantitative accuracy, and is restricted to usage for low-accuracy measurement items to perform a measurement. Further, when the parallel side faces of the development layer are opened, a state of the inspection target solution is affected, and in a case where blood components or the like, for example, are taken as the inspection target solution, a high-accuracy measurement becomes more difficult, resulting in restriction of a kind of the inspection target solution.

To solve these problems, Japanese Published Patent Application No. 2001-66310 discloses a chromatography quantitative measuring device in which the surface and parallel side faces of the development layer of the chromatography device are adherently covered with a liquid-impermeable sheet, so that a permeation state of the inspection target solution is arranged, thereby achieving a higher-accuracy measurement.

However, the chromatography device disclosed in Japanese Published Patent Application No. 2001-66310 requires a complicated operation because of an assembly method thereof, and an operation for sealing the parallel side faces at a part where the inspection target solution is applied becomes complicated in a case for example where a sufficiently thin membrane or the like is used for the development layer.

As a method for mass-producing the development layer of the immuno-chromatographic sensor, it is general that a reagent component is formed collectively on the development layer and the development layer is cut to be separated finally. When the immuno-chromatographic sensor as the chromatography device disclosed in Japanese Published Patent Application No. 2001-66310 is manufactured by this method, the parallel side faces cannot be sealed, and thus the cut immuno-chromatographic sensor has to be manufactured individually so as to seal its parallel side faces. Further, there are required a complicated operation to individually seal the parallel side faces with the liquid-impermeable sheet such as tape as well as materials to achieve that constitution, resulting in a high cost.

Further, in the above-described method of forming the development layer collectively, a reagent component is formed in a manner of crossing the direction in which the inspection target solution is developed in the development layer. In the immuno-chromatographic sensor which is formed by the above-described method and has its parallel side faces opened, when ununiform permeation of the inspection target solution in the development layer occurs, the flow rate at parts of the parallel side faces of the development layer is likely to change, and as a result, the amount of the marked reagent and measurement target in the inspection target solution passing through the reagent immobilization part are changed, so that the amount of the marked reagent component which performs bonding in the reagent immobilization part becomes ununiform over at the center part of the reagent immobilization part and at parts of the parallel side faces, resulting in reduced measurement accuracy. These problems are caused by employing a cutting tool such as a cutter, scissors, a mold press, a guillotine cutter, a rotary cutter, a score cutter and the like, as a cutting technique in a manufacturing method including a process of cutting the development layer, such as the manufacturing method of forming the development layer collectively. That is, the cutting tool should contact the development layer to cut the same, and a material which is changed in shape due to contact pressure, such as a nonwoven fabric, a glass fiber, a cellulose fiber, a membrane or the like, is mostly used for the development layer in general, so that the change in shape becomes ununiform at cutting, resulting in ununiform permeation in the measurement.

The present invention is made to solve the above-mentioned problems and has for its object to provide a high-accuracy biosensor which can arrange the permeation of the inspection target solution in the development layer and is easily manufactured at low cost, and a manufacturing method thereof.

According to an aspect of the present invention, there is provided a biosensor which is provided with a development layer for developing an inspection target solution, includes a region of an immobilized reagent which is immobilized in a part of the development layer that includes its side faces parallel to the direction of the inspection target solution permeating and a region of a marked reagent which is held in a part of the development layer that includes its side faces parallel to the direction of the inspection target solution permeating, in a marked dry state to be eluted by development of the inspection target solution, and measures a bonding amount of the marked reagent in the immobilized reagent region, thereby qualitatively or quantitatively measuring a measurement component in the inspection target solution, and in this biosensor, a reagent component on the side faces of the development layer parallel to the inspection target solution permeating, in the marked reagent region and the immobilized reagent region is denatured to be deactivated.

Therefore, it is possible to provide a biosensor which can prevent deterioration in measurement accuracy in the immobilized reagent region, which is due to ununiform permeation of the inspection target solution on the side faces of the development layer.

Preferably, the side faces of the development layer parallel to the direction of the inspection target solution permeating are partially or entirely melted and cured to be sealed.

Therefore, it is possible to provide a higher-accuracy biosensor which can prevent the deterioration in measurement accuracy in the immobilized reagent region, which is due to ununiform permeation of the inspection target solution on the side faces of the development layer, and arrange the permeation of the inspection target solution to enable a more accurate quantitative measurement. Further, it is possible to provide a biosensor which requires no new material for sealing the parallel side faces and enables simplification of a manufacturing process thereof, thereby reducing the cost owing to reduction in material and manufacturing process.

Preferably, in the biosensor as defined in any of Claims 1 to 3, the surface or surface and rear face of the development layer except for a part for applying the inspection target solution is covered with a liquid-impermeable sheet, and the side faces of the development layer and liquid-impermeable sheet parallel to the direction of the inspection target solution permeating are partially or entirely melted and cured to be sealed.

Therefore, the surface, rear face and parallel side faces of the development layer are sealed to prevent erroneous application of the inspection target solution, thereby providing a highly accurate biosensor which enables a highly accurate measurement.

Preferably, in the biosensor as defined in any of Claims 1 to 3, the surface or surface and rear face of the development layer except for parts at the beginning and end in the direction of the inspection target solution being applied is covered with a liquid-impermeable sheet, and the side faces of the development layer and liquid-impermeable sheet parallel to the direction of the inspection target solution permeating are partially or entirely melted and cured to be sealed.

Therefore, the surface and rear face of the development layer are covered with the liquid-impermeable sheet material and the parallel side faces are melted and cured to be sealed, so that liquid is intercepted, thereby preventing erroneous application of the inspection target solution, resulting in a highly accurate measurement. Further, the end of the development layer in the direction of the development of the inspection target solution is opened, whereby moisture is evaporated and, in relation to a water head difference between the inspection target solution in the end and the inspection target solution remaining in the application part, the inspection target solution continues to permeate in the direction of the downstream until the inspection target solution is dried, thereby requiring no absorbing member for absorbing extra inspection target solution, resulting in a simpler, low-cost and highly-accurate biosensor.

Preferably, in the biosensor as defined in any of Claims 1 to 3, the development layer is composed of a nitrocellulose.

Therefore, a biosensor which can be created easily can be provided.

Preferably, the parallel side faces of the development layer are melted by a laser and cured so as to be sealed.

Therefore, it is possible to provide a higher-accuracy biosensor in which a required part of the parallel side faces of the development layer can be melted and cured more speedily and uniformly and the melted and cured part is given uniformity.

According to Claim 8 of the present invention, in the biosensor as defined in any of Claims 1 to 3, the whole sensor including the immobilized reagent and the marked reagent is in a dry state.

Therefore, it is possible to provide a biosensor which is excellent in storage stability and is freely carried.

Preferably, the biosensor is an immuno-chromatography specimen in which a complex of the immobilized reagent and the marked reagent is formed on a permeable porous material, thereby performing a measurement.

Therefore, it is possible to provide a high-accuracy and precision immuno-chromatography as an immuno-chromatography which is prevailing as a simplified method in the market.

Preferably, the biosensor is a one-step immuno-chromatography specimen in which a measurement is performed on a permeable porous material by an applying operation of the inspection target solution.

Therefore, it is possible to provide a high-accuracy and precision one-step immuno-chromatography as a one-step immuno-chromatography which is prevailing as a simplified immunoassay method in the market.

Further the immobilized reagent and the marked reagent are formed on the same plane and member.

Therefore, it is possible to provide a highly accurate biosensor which is constituted by fewer materials to lower the cost as well as simplify the constitution and reduces variation in measurement values owing to the simple constitution so as to improve measurement accuracy.

Preferably, there is provided method for manufacturing a biosensor which is provided with a development layer for developing an inspection target solution, includes a region of an immobilized reagent which is immobilized in a part of the development layer and a region of a marked reagent which is held in a part of the development layer in a marked dry state to be eluted by development of the inspection target solution, and measures a bonding amount of the marked reagent in the immobilized reagent region, thereby qualitatively or quantitatively measuring a measurement component in the inspection target solution, and this biosensor manufacturing method includes a melting process for melting and curing side faces of the development layer which are parallel to the direction of the inspection target solution permeating to seal the same partially or entirely.

Therefore, the parallel side faces of the development layer itself are melted and cured to be sealed, so that no new material is required to seal the side faces, thereby creating a lower-cost biosensor.

Furthermore, the melting process is implemented by irradiating a laser to the side faces of the development layer partially or entirely.

Therefore, the melting and curing can be performed in no physical contact with the side faces of the development layer, thereby creating a high-accuracy biosensor in which the shape of the development layer is not changed due to contact pressure.

Preferably, there is provided a method for manufacturing a biosensor which is provided with a development layer for developing an inspection target solution, includes a region of an immobilized reagent which is immobilized in a part of the development layer and a region of a marked reagent which is held in a part of the development layer in a marked dry state to be eluted by development of the inspection target solution, and measures a bonding amount of the marked reagent in the immobilized reagent region, thereby qualitatively or quantitatively measuring a measurement component in the inspection target solution, and this biosensor manufacturing method includes a cutting and melting process for cutting the sheet-shaped development layer in parallel to the direction of the inspection target solution permeating, and simultaneously melting and curing cut surfaces of the development layer to seal.

Therefore, the sheet-shaped development layer is cut while its side faces are melted and cured at the same time, thereby creating a higher-accuracy, simplified and low-cost biosensor which requires no new sealing operation.

Furthermore, the cutting and melting process is implemented by trimming the sheet-shaped development layer by a laser.

Therefore, the parallel side faces of the development layer are melted and cured to be sealed in the process of trimming the sheet-shaped development layer by a laser, thereby creating a higher-accuracy, simplified and low-cost biosensor which requires no new sealing operation. Further, the melting and curing can be performed in no physical contact with the side faces of the development layer, thereby creating a high-accuracy biosensor in which the shape of the development layer is not changed due to contact pressure.

According to a second aspect of the present invention, there is provided a method for manufacturing a biosensor which is provided with a development layer for developing an inspection target solution, includes a region of an immobilized reagent which is immobilized in a part of the development layer that includes its side faces parallel to the direction of the inspection target solution permeating and a region of a marked reagent which is held in a part of the development layer that includes its side faces parallel to the direction of the inspection target solution permeating, in a marked dry state to be eluted by development of the inspection target solution, and measures a bonding amount of the marked reagent in the immobilized reagent region, thereby qualitatively or quantitatively measuring a measurement component in the inspection target solution, and this biosensor manufacturing method includes a denaturing and deactivating process for denaturing and deactivating a reagent component on the side faces parallel to the inspection target solution permeating, in the marked reagent region and the immobilized reagent region.

Therefore, the reagent component in the vicinity of the parallel side faces of the development layer can be denatured to be deactivated, thereby creating a biosensor which prevents deterioration in measurement accuracy due to ununiform permeation of the inspection target solution on the side faces of development layer

Preferably, the denaturing and deactivating process is implemented by irradiating a laser to the side faces.

Therefore, the reagent component can be denatured to be deactivated in no contact with the parallel side faces of the development layer, resulting in a higher-accuracy biosensor.

Preferably, there is provided a method for manufacturing a biosensor which is provided with a development layer for developing an inspection target solution, includes a region of an immobilized reagent which is immobilized in a part of the development layer that includes its side faces parallel to the direction of the inspection target solution permeating and a region of a marked reagent which is held in a part of the development layer that includes its side faces parallel to the inspection target solution permeating, in a marked dry state to be eluted by development of the inspection target solution, and measures a bonding amount of the marked reagent in the immobilized reagent region, thereby qualitatively or quantitatively measuring a measurement component in the inspection target solution, and this biosensor manufacturing method includes a melting and deactivating process for melting and curing side faces of the development layer which are parallel to the direction of the inspection target solution permeating to seal the same partially or entirely, as well as denaturing and deactivating a reagent component on the side faces parallel to the inspection target solution permeating, in the marked reagent region and the immobilized reagent region.

Therefore, it is possible to melt and cure the development layer to seal as well as denature and deactivate the reagent component on the parallel side faces in the same process, thereby creating a simpler and high-accuracy biosensor which requires only one operation.

Preferably, the melting and deactivating process is implemented by irradiating a laser to the side faces

Therefore, the melting and curing can be performed in no physical contact with the side faces of the development layer, thereby creating a high-accuracy biosensor in which the shape of the development layer is not changed due to contact pressure.

Preferably, there is provided a method for manufacturing a biosensor which is provided with a development layer for developing an inspection target solution, includes a region of an immobilized reagent which is immobilized in a part of the development layer that includes its side faces parallel to the direction of the inspection target solution permeating and a region of a marked reagent which is held in a part of the development layer that includes its side faces parallel to the inspection target solution permeating, in a marked dry state to be eluted by development of the inspection target solution, and measures a bonding amount of the marked reagent in the immobilized reagent region, thereby qualitatively or quantitatively measuring a measurement component in the inspection target solution, and this biosensor manufacturing method includes a cutting, melting and deactivating process for cutting the sheet-shaped development layer in parallel to the direction of the inspection target solution permeating, simultaneously melting and curing cut surfaces of the development layer to seal, and denaturing and deactivating a reagent component on the side faces in the marked reagent region and the immobilized reagent region.

Therefore, it is possible that the development layer is cut as well as melted and cured simultaneously to be sealed at the same time, and further the reagent component on the parallel side faces is denatured to be deactivated in the same process, thereby creating a simpler, low-cost and high-accuracy biosensor which can be cut, melted and denatured to be deactivated in a single operation.

Preferably, the cutting, melting and deactivating process is implemented by trimming the sheet-shaped development layer by a laser.

Therefore, it is possible that the development layer is cut as well as melted and cured simultaneously to be sealed at the same time, and further the reagent component on the parallel side faces is denatured to be deactivated in the same process, thereby creating a higher-accuracy, simple and low-cost biosensor which can be cut, melted and denatured to be deactivated in a single operation. Further, the melting and curing can be performed in no physical contact with the side faces of the development layer, thereby creating a high-accuracy biosensor in which the shape of the development layer is not changed due to contact pressure.

### BRIEF DESCRIPTION OF DRAWINGS

Figures 1 are perspective views illustrating constitutions of a biosensor according to a first embodiment of the present invention.
Figures 2 are perspective views illustrating constitutions of a biosensor according to a second embodiment of the present invention.
Figures 3 are perspective views illustrating constitutions of a biosensor according to third embodiment of the present invention.
Figures 4 are diagrams illustrating constitutions of a cross section of a biosensor before and after cutting, in which a reagent holding part is not in contact with parallel side faces of a development layer according to a fourth embodiment of the present invention.
Figures 5 are diagrams illustrating constitutions of a cross section of a biosensor before and after cutting, in which the reagent holding part is in contact with the parallel side faces of the development layer according to the fourth embodiment of the invention.
Figure 6 illustrates a micrograph of a cross section of a biosensor which is cut in a conventional cutting process.
Figure 7 illustrates a micrograph of a cross section of a biosensor which is cut in a cutting process according to the fourth embodiment of the invention.
Figures 8 are perspective views illustrating constitutions of a biosensor according to a fifth embodiment of the present invention.
Figures 9 are pattern diagrams illustrating measurement states of the biosensor according to the fifth embodiment of the invention.
Figures 10 are graphs illustrating relationships between hCG concentration and a measurement result in two kinds of immuno-chromatographic specimens according to an example of the present invention.

### BEST MODE TO EXECUTE THE INVENTION

Hereinafter, embodiments of the present invention will be described.

### (Embodiment 1)

A biosensor and a manufacturing method thereof according to a first embodiment of the present invention make an inspection target solution permeate on a development layer at a uniform rate.

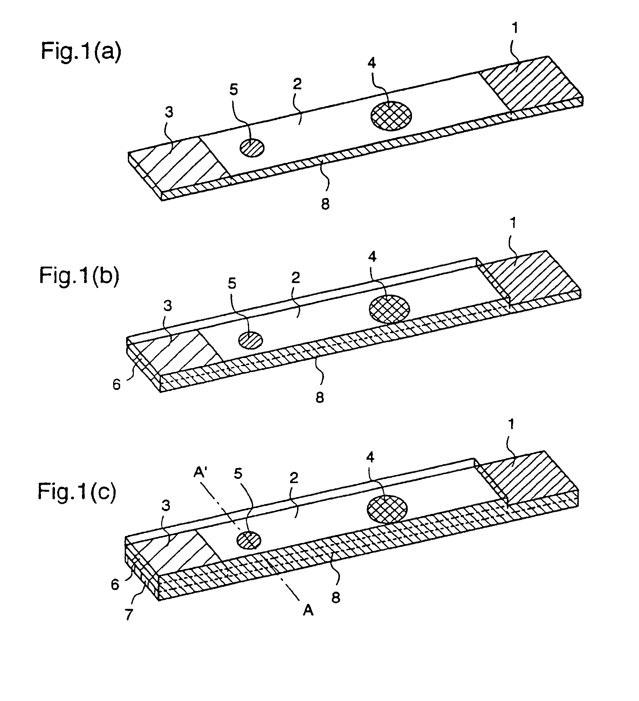

First, the constitution of the biosensor according to the first embodiment will be described with reference to figures 1.

Figures 1 are perspectives views illustrating constitutions of the biosensor which is sealed by melting and curing; figure 1(a) illustrates the biosensor, which is constituted only by the development layer, figure 1(b) illustrates the biosensor, which is provided with a liquid-impermeable sheet material on the development layer, and figure 1(c) illustrates the biosensor, which is provided with the liquid-impermeable sheet material on the development layer and a base material thereunder.

In figures 1, numeral 1 denotes an application part 1 for applying an inspection target solution onto the development layer, which is composed of a nonwoven fabric. Numeral 2 denotes a reaction part in the reactive layer, which is composed of a nitrocellulose. Numeral 3 denotes a water absorbing part for absorbing the solution permeating on the development layer, which is composed of glass fiber filter paper. These materials used for the development layer may be arbitrary porous materials which can be permeated by the inspection target solution, such as filter paper, a nonwoven fabric, a membrane, a fabric or the like.

Numeral 4 denotes a marked reagent holding part at a part on the development layer, in which an antibody for a measurement target in the inspection target solution is held, being marked with a gold colloid, in a dry state so as to be eluted by the inspection target solution. Numeral 5 denotes a reagent immobilization part, in which the antibody for the measurement target in the inspection target solution is immobilized in a dry state so as to be bonded with a different epitope as that for the marked reagent to form a complex of the immobilized antibody, the measurement target in the inspection target solution and the marked reagent. In the first embodiment, the marked reagent holding part 4 and the reagent immobilization part 5 are formed in spot shapes at parts on the development layer, and particularly constituted to prevent the reagent from being in contact with side faces which are parallel to the direction of the inspection target solution permeating. While the marked reagent holding part 4 and the reagent immobilization part 5 have the spot shapes, they do not necessarily have the spot shapes, and any shapes can be selected freely as long as the marked reagent holding part 4 and the reagent immobilization part 5 are formed in shapes which prevent their contacts with the parallel side faces of the development layer. Further, while the development layer having the marked reagent holding part 4 and the reagent immobilization part 5 is constituted to cause a so-called sandwich reaction in the antigen antibody reaction, the development layer may be constituted to cause a competitive reaction when a reagent which competitively reacts to the measurement target in the inspection target solution is employed according to selection of the reagent. Further, when a specific bonding other than a bonding caused by the antigen antibody reaction is to be utilized, the development layer can be constituted by an arbitrary reagent component. With respect to a marking method, the above-described gold colloid is only an example, and others, such as an enzyme, a protein, coloring matters, a fluorescene, a metallic sol, a nonmetallic sol, and coloring particles such as a latex, can be selected arbitrarily according to need.

Numeral 6 denotes a liquid-permeable sheet material, which is composed of transparent PET tape. This liquid-permeable sheet material 6 adherently covers the development layer except for the application part 1. The liquid-permeable sheet material 6 covers the above-described part of the development layer, thereby protectingly intercepting application of the inspection target solution to parts other than the application part 1 as well as preventing external pollution due to the inspection target solution being improvidently in contact with the development layer or an inspector directly touching the development layer with his/her hand or the like. It is preferred that a transparent material is employed for this liquid-permeable sheet material 6, and particularly a part for covering the reagent immobilization part 5, which is a part for confirming a measurement result, is at least in a permeable state. In a case where the biosensor does not require a high-accuracy result or the formed development layer is finally put in a hollow casing, the liquid-permeable sheet material 6 is not indispensable.

Numeral 7 denotes a base material for holding the development layer, which is composed of a white PET film, for example. The base material 7 reinforces the development layer, and when a solution which poses a risk of infection, such as blood, saliva, and urine, is employed as the inspection target solution, the base material 7 intercepts it. Further, it is also possible that the base material 7 intercepts light in a case where the development layer is permeated and becomes light permeable. While the base material 7 is arranged under the development layer here, the liquid-permeable sheet material 6, instead of the base material 7, may cover the rear face of the development layer. When the solution which poses a risk of infection, such as blood, saliva, and urine, is employed as the inspection target solution, that solution is intercepted also in this case, and the number of materials of the biosensor can be decreased, thereby further reducing the cost.

Numeral 8 denotes a melted and cured part, which is formed by melting the parallel side faces of the development layer by a CO₂ laser and thereafter curing the same by cooling. Since in the first embodiment the marked reagent holding part 4 and the reagent immobilization part 5 are not in contact with the parallel side faces of the development layer, the marked reagent holding part 4 and the reagent immobilization part 5 are not affected even when the side faces thereof are melted. Therefore, the amount of the reagent in the marked reagent holding part 4 and the reagent immobilization part 5 can be minimized, and this is effective when the reagent is expensive or minute in amount. Further, while in the first embodiment the CO₂ laser is employed as a melting method, others such as an excimer laser, an argon laser, a YAG laser, a helium neon laser, a ruby laser and the like can be also employed. In addition to the laser, the parallel side faces of the development layer can be also melted by bringing a heated metal or the like into contact therewith, as well as by an organic solvent or the like depending on a material of the development layer. The above-described melting methods are only examples, and an arbitrary method is available as long as the parallel side faces of the development layer can be melted.

Next, a measuring method of the so-constituted biosensor according to the first embodiment will be described with reference to figures 1. The biosensor exemplified in the first embodiment is a specimen for a one-step immuno-chromatography, and a basic measurement operation using the biosensor comprises only one operation of applying the inspection target solution so as to start a measurement. The immuno-chromatography here is an immunoassay method in which a permeable porous material is employed and a complex of the immobilized reagent and the marked reagent is formed, thereby performing a measurement, and is a system of measurement that utilizes the antigen antibody reaction, in which B/F separation is implemented in the process of the inspection target solution permeating a chromatography carrier, while a washing operation such as the B/F separation is required in a usual immunoassay method. In this immuno-chromatography, whole reagent is usually in a dry state, and a marker for marking the reagent is usually a gold colloid or a latex, while magnetic particles, an enzyme, a metallic colloid or the like are also used. When the marker is an enzyme or the like, there is included an operation of adding an enzyme substrate or a reaction stop reagent as a measurement operation by a user.

A measurement by the so-constituted biosensor starts when an appropriate amount of inspection target solution is applied to the application part 1. When the inspection target solution is applied to the application part 1, the inspection target solution permeates the development layer. Since the development layer has its side faces sealed with the melted and cured part 8, a permeation rate of the inspection target solution at the side faces is not increased, resulting in permeation at a uniform rate. Then, when the inspection target solution reaches to the marked reagent holding part 4, the marked reagent held in the marked reagent holding part 4 starts to be eluted. Thereafter, the permeation of the inspection target solution being promoted, the inspection target solution reaches to the reagent immobilization part 5 and then absorbed in the water absorbing part 3.

Then, the measurement result is obtained by confirming a bonding state of the marked reagent in the reagent immobilization part 5 in a predetermined period of time. Since in the first embodiment the marked reagent holding part 4 and the reagent immobilization part 5 are not in contact with the parallel side faces of the development layer and the parallel side faces are melted to be sealed, uniform permeation of the inspection target solution is performed in the reagent immobilization part 5, whereby the marked reagent and the measurement target in the inspection target solution, which pass through the reagent immobilization part 5, permeate respective surfaces of the reagent immobilization part 5 uniformly, and thus a partial difference in the bonding amount is eliminated, so that a highly accurate measurement result can be obtained.

Further, the bonding state of the marked reagent in the reagent immobilization part 5 can be measured visually when a qualitative judgement is required. When a more accurate measurement is required, the bonding amount of the marked reagent is measured employing an optical method, thereby obtaining a quantitative result. Further, since in the first embodiment the reagent immobilization part 5 can be shaped so that an inspector easily confirms the result, a visual judgement or the like is suitable as a method of measuring the boding state of the marked reagent in the reagent immobilization part 5.

As described above, according to the biosensor and the manufacturing method thereof in the first embodiment, the marked reagent holding part 4 and the reagent immobilization part 5 are formed in spot shapes at parts on the development layer so as not to be in contact with its parallel side faces, and the parallel side faces are partially or entirely malted and cured so as to be sealed in the direction of the inspection target solution on the development layer permeating, whereby the permeation of the inspection target solution in the development layer can be arranged, resulting in a more accurate quantitative measurement. Further, since the development layer itself is melted and cured, no new material is required for sealing the parallel side faces, and a manufacturing process can be simplified, thereby reducing the cost due to reduction in material and manufacturing process.

Further, a nitrocellulose, which makes bonding of a protein or the like relatively easy, is employed as a material of the development layer, thereby making it easy to manufacture the biosensor.

Further, the parallel side faces of the development layer are melted by a laser and cured so as to be sealed, whereby a required part of the parallel side faces can be melted and cured more speedily and uniformly and the melted and cured part can be given uniformity. Further, the side faces of the development layer can be melted and cured in a no-physical-contact state, so that the shape of the development layer is not changed due to contact pressure, resulting in a high-accuracy measurement.

While in the first embodiment the biosensor utilizing the antigen antibody reaction has been given as an example, any specific bonding reactions are similarly available as well as the antigen antibody reaction. In addition, the measuring operation is not restricted to one which comprises one-step operation of applying the inspection target solution so as to perform a measurement, and one which requires plural operations, such as applying a reaction stop reagent or the like when the marker is an enzyme, or diluting the specimen, in addition to applying the inspection target solution, is also available.

### (Embodiment 2)

In a biosensor and a manufacturing method thereof according to a second embodiment of the present invention, a reagent component on side faces parallel to the direction in which an inspection target solution permeates on the development layer, in the marked reagent holding part 4 and the reagent immobilization part 5 is denatured to be deactivated.

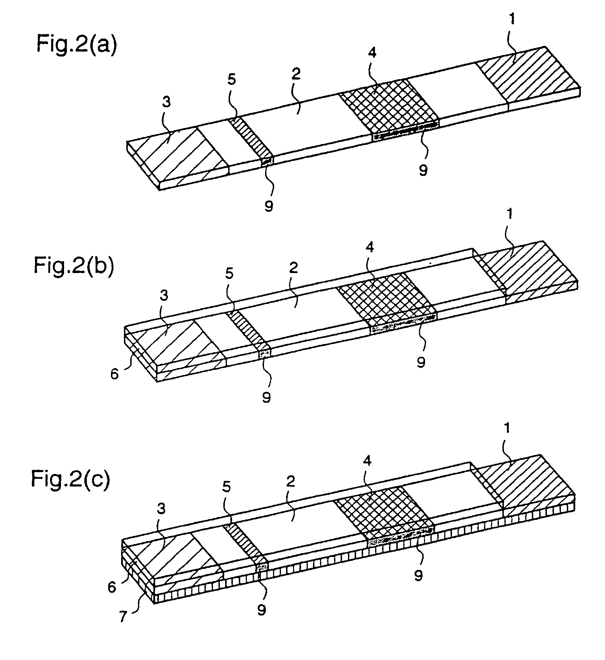

First, the constitution of the biosensor according to the second embodiment will be described with reference to figures 2.

Figures 2 are perspective views illustrating the biosensor in a state where the reagent component on the parallel side faces of the development layer are denatured to be deactivated; figure 2(a) illustrates the biosensor, which is constituted only by the development layer, figure 2(b) illustrates the biosensor, which is provided with a liquid-impermeable sheet material on the development layer, and figure 2(c) illustrates the biosensor, which is provided with the liquid-impermeable sheet material on the development layer and a base material thereunder.

In figures 2, the biosensor according to the second embodiment is a specimen for the one-step immuno-chromatography as in the first embodiment, in which the marked reagent holding part 4 and the reagent immobilization part 5 are formed on the development layer. In this embodiment, the marked reagent holding part 4 and the reagent immobilization part 5 are formed in strip shapes and the reagent is in contact with the side faces parallel to the direction in which the inspection target solution permeates on the development layer. Numeral 9 denotes a reagent deactivation part, which is obtained by deactivating side faces of the development layer in the marked reagent holding part 4 and in the reagent immobilization part 5 by a CO₂ laser. To deactivate the reagent component on the side faces of the development layer, a method of uniformly deactivating the vicinity of the side faces, such as a method of deactivating by bringing a heated metal surface into contact with the side faces or a method of applying and atomizing a solution of acid, alkali or the like which denatures a protein, is preferred. In figures 2, the same parts as those shown in figures 1 are denoted by the same reference numerals and their descriptions will be omitted.

Next, a measuring method of the so-constituted biosensor according to the second embodiment will be described with reference to figures 2.

First, a measurement by the above-described biosensor starts when an appropriate amount of inspection target solution is applied to the application part 1. When the inspection target solution is applied to the application part 1, the inspection target solution permeates on the development layer. When the inspection target solution permeates the marked reagent holding part 4, the marked reagent starts to be eluted. However, the marked reagent on the side faces of the development layer in the marked reagent holding part 4 is deactivated, and therefore that marked reagent is not eluted or has lost its specific property. The specific property here is for example a specific bonding reaction such as the antigen antibody reaction or a specific reaction in the case of employing an enzyme or the like as the marked reagent in the marked reagent holding part 4.

Subsequently, the permeation of the inspection target solution in the development layer being promoted, the inspection target solution reaches to the reagent immobilization part 5 and then absorbed in the water absorbing part 3. Also in this reagent immobilization part 5, the reagent on the side faces of the development layer is denatured to be deactivated, and thus no reaction is caused on the side faces of the reagent immobilization part 5.

The permeation of the inspection target solution in the development layer employs no mechanical operation and thus is performed according to the property of the development layer. Considering that fine pores in the development layer are cut into pieces on the side faces of the development layer and their shapes are different from those at the center part, a permeation rate varies between on the both side faces of the development layer and at the center part. Therefore, in the second embodiment, the reagent on the side faces of the development layer is denatured to be deactivated so that no reaction is caused on the side faces of the development layer as described above, thereby eliminating a bonding of the marked reagent on the side faces of the reagent immobilization part 5 where the permeation of the inspection target solution is ununiform. There is a large number of fine pores here, and the center part of the development layer indicates parts other than the side faces.

Then, the measurement result of the above-described biosensor is obtained by confirming a bonding state of the marked reagent in the reagent immobilization part 5 in a predetermined period of time. The bonding state of the marked reagent in this reagent immobilization part 5 can be measured visually when a qualitative judgement is required. When a more accurate measurement is required, the bonding amount of the marked reagent is measured employing an optical method, thereby obtaining a quantitative result.

As described above, according to the biosensor and the manufacturing method thereof in the second embodiment, the marked reagent holding part 4 and the reagent immobilization part 5 are formed on the development layer in strip shapes, and the parallel side faces of the marked reagent holding part 4 and reagent immobilization part 5 on the development layer are denatured to be deactivated, so that no reaction is caused on the side faces of the development layer, thereby preventing deterioration in measurement accuracy due to ununiform permeation of the inspection target solution.

Further, the reagent deactivation part 9 of the development layer is obtained by denaturing and deactivating the reagent component in no contact therewith by laser irradiation, whereby the shape of the development layer is not changed due to contact pressure in the denaturing and deactivating process, so that ununiform permeation due to the change in the shape of the development layer shape can be prevented, resulting in manufacture of high-accuracy biosensor.

### (Embodiment 3)

A biosensor and a manufacturing method thereof according to a third embodiment of the present invention make an inspection target solution permeate on a development layer at a uniform rate, in which side faces of the development layer parallel to the direction of the inspection target solution permeating are melted and cured to be sealed, and at the same time, a reagent component on the parallel side faces of the marked reagent holding part 4 and the reagent immobilization part 5 which are formed on the development layer in strip shapes is denatured to be deactivated.

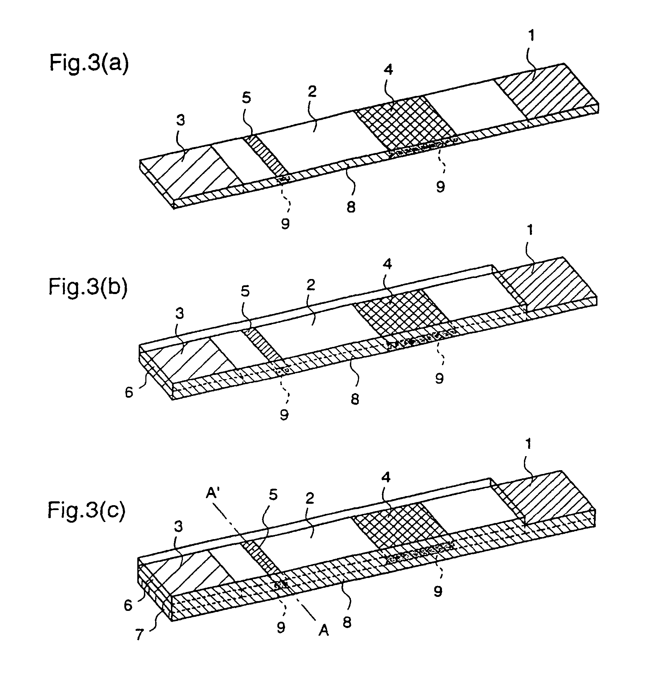

First, the constitution of the biosensor according to the third embodiment will be described with reference to figures 3.

Figures 3 are perspectives views illustrating constitutions of the biosensor in which parallel side faces of the development layer are sealed by melting and curing and the reagent component thereon is denatured to be deactivated; figure 3(a) illustrates the biosensor, which is constituted only by the development layer, figure 3(b) illustrates the biosensor, which is provided with a liquid-impermeable sheet material on the development layer, and figure 3(c) illustrates the biosensor, which is provided with the liquid-impermeable sheet material on the development layer and a base material thereunder.

In figures 3, in the biosensor according to the third embodiment, the marked reagent holding part 4 and the reagent immobilization part 5 are formed in strip shapes as in the second embodiment, and the side faces parallel to the direction in which the inspection target solution is applied on the development layer are entirely melted by irradiating a CO₂ laser or the like and cured so as to be sealed (melted and cured part 8), and at the same time, the reagent component on the parallel side faces is denatured to be deactivated (reagent deactivation part 9). In figures 3, the same parts as those shown in figures 2 are denoted by the same reference numerals and their descriptions will be omitted.

Next, a measuring method of the so-constituted biosensor according to the third embodiment will be described with reference to figures 3.

First, a measurement by the above-described biosensor starts when an appropriate amount of inspection target solution is applied to the application part 1. When the inspection target solution is applied to the application part 1, the inspection target solution permeates on the development layer. Since the development layer has its side faces sealed with the melted and cured part 8, a permeation rate at the side faces is not increased, resulting in permeation at a uniform rate. Then, when the inspection target solution permeates the marked reagent holding part 4, the marked reagent starts to be eluted. However, the marked reagent on the side faces of the development layer in the marked reagent holding part 4 is deactivated, and therefore that marked reagent is not eluted or has lost its specific property. The specific property here is for example a specific bonding reaction such as the antigen antibody reaction or a specific reaction in the case of employing an enzyme or the like as the marked reagent in the marked reagent holding part 4.

Subsequently, the permeation of the inspection target solution in the development layer being promoted, the inspection target solution reaches to the reagent immobilization part 5 and then absorbed in the water absorbing part 3. Also in this reagent immobilization part 5, the reagent on the side faces of the development layer is denatured to be deactivated, and thus no reaction is caused on the side faces of the reagent immobilization part 5.

The measurement result of this biosensor can be obtained by confirming a bonding state of the marked reagent in the reagent immobilization part 5 in a predetermined period of time. Since in the third embodiment the parallel side faces of the development layer are melted to be sealed, uniform permeation of the inspection target solution is performed in the reagent immobilization part 5, whereby the marked reagent and the measurement target in the inspection target solution, which pass through the reagent immobilization part 5, permeate respective surfaces of the reagent immobilization part 5 uniformly, and thus a partial difference in the bonding amount is eliminated, resulting in a highly accurate measurement result. Further, in the third embodiment, the parallel side faces of the development layer in the marked reagent holding part 4 and the reagent immobilization part 5 are denatured to be deactivated, whereby ununiform permeation of the inspection target solution in the reagent immobilization part 5 which is due to variation in a permeation rate between on the side faces of the development layer and at the center part can be eliminated by preventing the bonding of the marked reagent on the side faces of the reagent immobilization part 5, resulting in a more accurate and uniform measurement result. The center part of the development layer here indicates parts other than the side faces. Further, the bonding state of the marked reagent in the reagent immobilization part 5 can be measured visually when a qualitative judgement is required. When a more accurate measurement is required, the bonding amount of the marked reagent is measured employing an optical method, thereby obtaining a quantitative result.

As described above, according to the biosensor and the manufacturing method thereof in the third embodiment, the marked reagent holding part 4 and the reagent immobilization part 5 are formed on the development layer in strip shapes, and the side faces parallel to the direction in which the inspection target solution permeates on the development layer are partially or entirely melted and cured so as to be sealed, and at the same time, the reagent component on the parallel side faces in the marked reagent holding part 4 and the reagent immobilization part 5 is denatured to be deactivated, whereby the permeation of the inspection target solution into the development layer is arranged and deterioration in accuracy due to the permeation into the parallel side faces in the reagent immobilization part 5 is eliminated, resulting in a more accurate quantitative measurement. Further, since the development layer itself is melted and cured, no new material is required for sealing the parallel side faces, and a manufacturing process can be simplified, thereby reducing the cost due to reduction in material and manufacturing process.

Further, a nitrocellulose, which makes bonding of a protein or the like relatively easy, is employed as a material of the development layer, thereby making it easy to manufacture the biosensor.

Further, the parallel side faces of the development layer are melted by a laser and cured so as to be sealed, whereby a required part of the parallel side faces can be melted and cured more speedily and uniformly and the melted and cured part can be given uniformity. Further, the side faces of the development layer can be melted and cured in a no-physical-contact state, so that the shape of the development layer is not changed due to contact pressure, resulting in a high-accuracy measurement.

### (Embodiment 4)

In a biosensor and a manufacturing method thereof according to a fourth embodiment of the present invention, the biosensor is cut and its cut surfaces are sealed in a single process at manufacture.

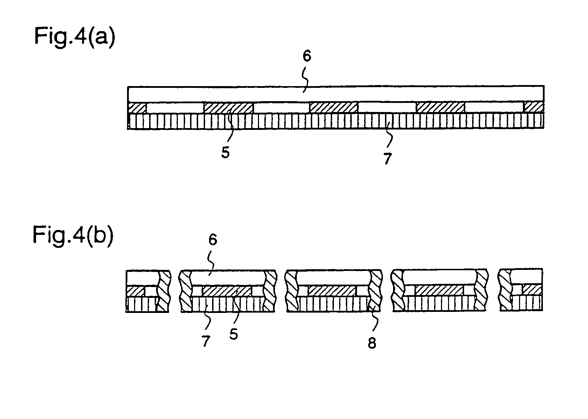

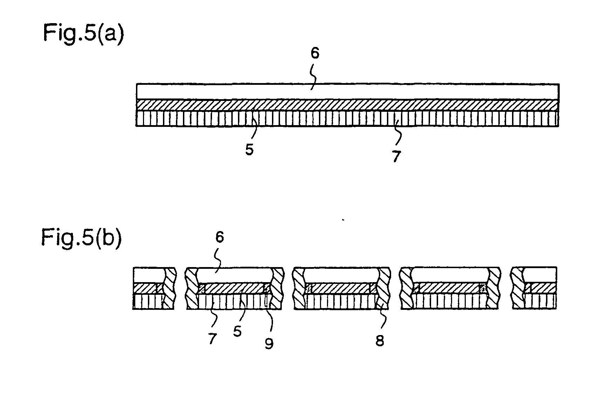

Hereinafter, a manufacturing process of the biosensor according to the fourth embodiment will be described with reference to figures 4 and 5.

Figures 4 are A-A' cross section diagrams before and after the cutting process of the biosensor that has a spot-shaped reagent immobilization part according to the fourth embodiment; figure 4(a) illustrates a state before cutting and figure 4(b) illustrates a state after cutting. Figures 5 are A-A' cross section diagrams before and after the cutting process of the biosensor that has a strip-shaped reagent immobilization part according to the fourth embodiment; figure 5(a) illustrates a state before cutting and figure 5(b) illustrates a state after cutting. Figures 4 and 5 illustrate A-A' cross sections in figure 1(c) and figure 3(c), and the same parts as those shown in figures 1 and 3 are denoted by the same reference numerals and their descriptions will be omitted.

In figure 4(a), the biosensor is formed in a sheet shape by the development layer, the liquid-impermeable sheet material 6 and the base material 7. The sheet shape here is a state before cutting where the biosensor continue plurally as shown in figure 4(b). In the fourth embodiment, the sheet-shaped biosensor in figure 4(a) is cut employing a CO₂ laser to manufacture plural biosensors as shown in figure 4(b) from one sheet-shaped biosensor. In the manufacturing process of the biosensor according to the fourth embodiment, when the sheet-shaped biosensor is cut, its sides faces are melted and cured to form the melted and cured part 8 at the same time as shown in figure 4(b), which means the cutting and the sealing of the cut surfaces can be performed in a single process.

Also in figure 5(a), the biosensor is formed in a sheet shape, and the sheet-shaped biosensor in figure 5(a) is cut employing a CO₂ laser here to manufacture plural biosensors as shown in figure 5(b) from one sheet-shaped biosensor. Since, in figures 5, the marked reagent holding part 4 and the reagent immobilization part 5 are formed in strip shapes, the cutting and the sealing of the cut surfaces, as well as the denaturation and deactivation of a reagent component on side faces of the marked reagent holding part 4 and the reagent immobilization part 5 are performed at the same time in a single process, which means the melted and cured part 8 and the reagent deactivation part 9 can be formed in the biosensor in a single cutting process.

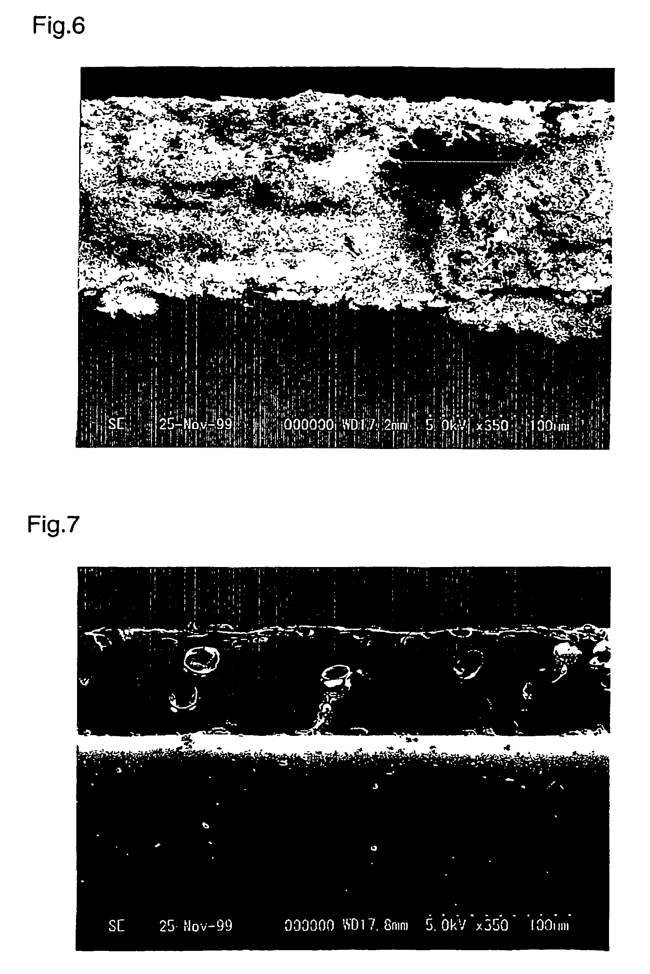

Here, a comparison will be made between a cross section state of the biosensor according to the manufacturing process of the fourth embodiment and a cross section state of a biosensor according to a conventional manufacturing process with reference to figures 6 and 7. Figure 6 illustrates a micrograph of the side faces parallel to the direction of the inspection target solution permeating in a case where the biosensor is cut with a conventional cutting method, and figure 7 illustrates a micrograph of the side faces parallel to the direction of the inspection target solution permeating in a case where the biosensor is cut by the cutting method according to the fourth embodiment.

The side faces of the biosensor manufactured by the manufacturing process of the fourth embodiment, in which the cutting employing a CO₂ laser and the melting and curing of the side faces are performed at the same time, are melted and cured to be sealed as shown in figure 7. On the other hand, the side faces of the conventional biosensor manufactured by the conventional manufacturing process are changed in shape as the side faces of the development layer are damaged by a cutting tool brought into contact therewith.

In comparison between figures 6 and 7, it is confirmed that the side faces of the development layer in figure 6 is deformed, while the side faces of the development layer in figure 7 are kept remaining porous and are melted and cured to be sealed.

As described above, according to the biosensor and the manufacturing method thereof in the fourth embodiment, the sheet-shaped biosensor is cut by a laser and the cut surfaces are melted and cured simultaneously to be sealed, thereby requiring no operation for sealing in a process different from the cutting process in the manufacturing process of the biosensor, resulting in a simple, low-cost, and high-accuracy biosensor.

### (Embodiment 5)

In a biosensor and a manufacturing method thereof according to a fifth embodiment of the present invention, a development layer is made of a single member, a water absorbing part or the like is eliminated, and a liquid-permeable sheet material at the beginning and end of the development layer is removed, in order to reduce the cost as well as simplify a manufacturing process with high accuracy and precision.

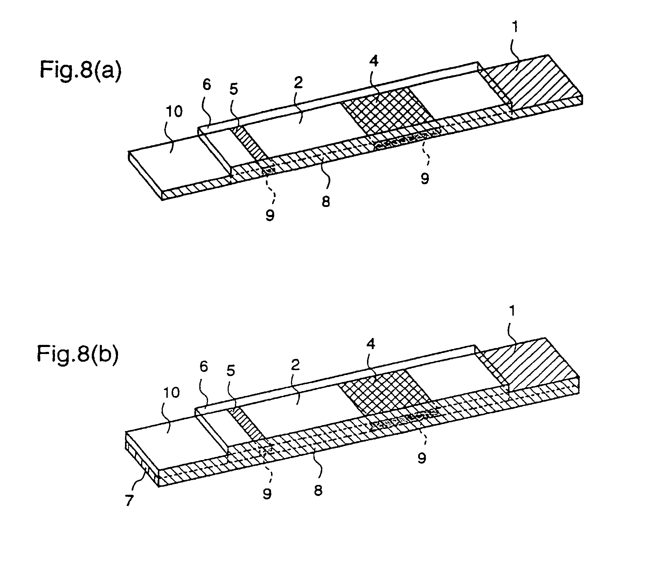

First, the constitution of the biosensor according to the fifth embodiment will be described with reference to figures 8.

Figures 8 are perspective views illustrating the biosensor which is sealed by melting and curing; figure 8(a) illustrates the biosensor, which is provided with the liquid-impermeable sheet material on the development layer, and figure 8(b) illustrates the biosensor, which is provided with the liquid-impermeable sheet material on the development layer and a base material thereunder.

In figures 8, numeral 1 denotes an application part for applying an inspection target solution onto the development layer, which is made of the same member as that of a reaction part 2. Numeral 2 denotes the reaction part in the development layer, which is composed of a nitrocellulose. Numeral 10 denotes an end open part in the development layer which is not covered with the liquid-permeable sheep material. These materials used for the development layer may be arbitrary porous materials which can be permeated by the inspection target solution, such as filter paper, a nonwoven fabric, a membrane, a fabric or the like.

In the case of aiming at reducing the cost of the biosensor as well as simplifying the manufacturing process thereof, it is preferred that the development layer is composed of a single member and further is made of a thick material since the inspection target solution is required to be developed in the development layer even when the inspection target solution is small in amount (100 µL or less) or is extremely small in amount (10 µL or less). Therefore, a membrane such as a nitrocellulose is preferable to be employed as the material of the development layer.

Numeral 4 denotes a marked reagent holding part at a part on the development layer, in which an antibody for a measurement target in the inspection target solution is held, being marked with a gold colloid, in a dry state so as to be eluted by the inspection target solution. Numeral 5 denotes a reagent immobilization part, in which the antibody for the measurement target in the inspection target solution is immobilized in a dry state so as to be bonded with a different epitope as that for the marked reagent to form a complex of the immobilized antibody, the measurement target in the inspection target solution and the marked reagent. Further, while the development layer is constituted to cause a so-called sandwich reaction in the antigen antibody reaction, a competitive reaction is also available when a reagent which competitively reacts to the measurement target in the inspection target solution is employed according to selection of the reagent. Further, when a specific bonding other than a bonding caused by the antigen antibody reaction is to be utilized, the development layer can be constituted by an arbitrary reagent component. With respect to a marking method, the above-described gold colloid is only an example, and others, such as an enzyme, a protein, coloring matters, a fluorescene, a metallic sol, a nonmetallic sol, and coloring particles such as a latex, can be selected arbitrarily according to need.

In the case of aiming at improving accuracy as well as reducing the cost of the biosensor and simplifying the manufacturing process thereof, it is preferred that the above-described both marked reagents are held or immobilized in the same member, the application part 1, the reaction part 2 and the water absorbing part 3 are not separated members but a single member, and the both reagent components are provided at different parts on the same plane.

Numeral 6 denotes a liquid-permeable sheet material, which is composed of transparent PET tape. This liquid-permeable sheet material 6 adherently covers the development layer except for the application part 1 and the end open part 10 of the development layer. The liquid-permeable sheet material 6 covers the above-described part of the development layer, thereby protectingly intercepting application of the inspection target solution to parts other than the application part 1 and the end open part 10 as well as preventing external pollution due to the inspection target solution being improvidently in contact with the development layer or an inspector directly touching the development layer with his/her hand or the like. It is preferred that a transparent material is employed for this liquid-permeable sheet material 6, and particularly a part for covering the reagent immobilization part 5, which is a part for confirming a measurement result, is at least in a permeable state. Further, it is preferable that the liquid-impermeable sheet material 6 adherently covers the development layer in order to obtain a high-accuracy measurement result by giving uniformity in permeation to the inspection target solution that is permeating the development layer.

Numeral 7 denotes a base material for holding the development layer, which is composed of a white PET film, for example. The base material 7 reinforces the development layer, and when a solution which poses a risk of infection, such as blood, saliva, and urine, is employed as the inspection target solution, the base material 7 intercepts it. Further, it is also possible that the base material 7 intercepts light in a case where the development layer is permeated and becomes light permeable.

Numeral 8 denotes a melted and cured part, which is formed by melting parallel side faces of the development layer by a CO₂ laser and thereafter curing the same by cooling. While the CO₂ laser is employed as a melting method here, others such as an excimer laser, an argon laser, a YAG laser, a helium neon laser, a ruby laser and the like can be also employed. In addition to the laser, the parallel side faces of the development layer can be also melted by bringing a heated metal or the like into contact therewith, as well as by an organic solvent or the like depending on a material of the development layer. The above-described melting methods are only examples, and an arbitrary method is available as long as the parallel side faces of the development layer can be melted.

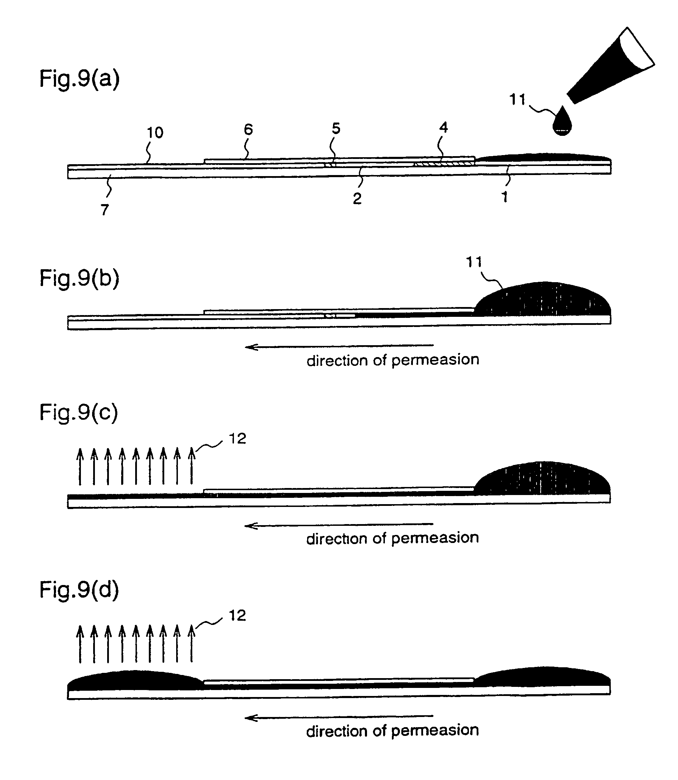

Next, a measuring method of the so-constituted biosensor according to the fifth embodiment will be described with reference to figures 9.

Figures 9 are pattern diagrams illustrating measurement states of the biosensor according to the fifth embodiment.

A measurement by the above-described biosensor is started when an appropriate amount of inspection target solution 11 is applied to the application part 1 (figure 9(a)). When the inspection target solution 11 is applied to the application part 1, the inspection target solution 11 permeates the development layer (figure 9(b)). Since the development layer has its side faces sealed with the melted and cured part 8, a permeation rate of the inspection target solution 11 at the side faces is not increased, resulting in permeation in the development layer at a uniform rate. Then, when the inspection target solution 11 reaches to the marked reagent holding part 4, the marked reagent held in the marked reagent holding part 4 starts to be eluted. Thereafter, the permeation of the inspection target solution 11 being promoted, the inspection target solution 11 reaches to the reagent immobilization part 5 and then to the end open part 10 (figure 9(c)). The inspection target solution 11 which reaches to the end open part 10 is dried by evaporation 12 and further exuded to the same height as that of the inspection target solution 11 in the application part 1 in relation to a water head difference (figure 9(d)).

Therefore, this biosensor does not require a new material for absorbing extra inspection target solution 11 and enables the inspection target solution 11 to permeate the development layer in the predetermined direction, from the direction of the inspection target solution 1 to the direction of the end open part 10. While in the fourth embodiment the application part 1 and the end open part 10 are exposed and opened, it is also possible according to need that the application part 1 and the end open part 10 are constituted by a protective material or that the development layer with the above-described constitution is put in a hollow casing so that the inspection target solution 11 is not attached to inspector's hand, in a case for example where the inspection target solution 11 is staining substance.

Then, the measurement result is obtained by confirming a bonding state of the marked reagent in the reagent immobilization part 5 in a predetermined period of time. Since in the present invention the parallel side faces of the development layer in the reagent immobilization part 5 are melted to be sealed as described above, uniform permeation of the inspection target solution 11 in the development layer is performed, whereby the marked reagent and the measurement target in the inspection target solution 11, which pass through the reagent immobilization part 5, permeate respective surfaces of the reagent immobilization part 5 uniformly, and thus a difference in the bonding amount of the marked reagent between at the center of the reagent immobilization part 5 and on the side faces is eliminated, resulting in a highly accurate measurement result.

The marked reagent in this reagent immobilization part 5 can be measured visually when a qualitative judgement is required. When a more accurate measurement is required, the bonding amount of the marked reagent in the reagent immobilization part 5 is measured employing an optical method, thereby obtaining a quantitative result.

While in the fifth embodiment a description has been given taking a case where the marked reagent holding part 4 and the reagent immobilization part 5 are formed on the development layer in strip shapes as an example, the marked reagent holding part 4 and the reagent immobilization part 5 may be formed also in spot shapes.

As described above, according to the biosensor and the manufacturing method thereof in the fifth embodiment, the side faces of the development layer parallel to the direction of the inspection target solution 11 permeating are partially or entirely melted and cured to be sealed, so that the permeation of the inspection target solution 11 is arranged, thereby enabling a more accurate quantitative measurement, and further the development layer except for its end and beginning is adherently covered with the liquid-permeable sheet material 6, whereby it is possible to eliminate the use of a member for the part for applying the inspection target solution and a member for the part for absorbing the inspection target solution.

Further, since the development layer itself is melted and cured, no new material is required for sealing the parallel side faces, and a manufacturing process can be simplified, whereby the cost can be reduced due to reduction in material and manufacturing process, while sensor performance is maintained.

Further, a nitrocellulose, which makes bonding of a protein or the like relatively easy, is employed as a material of the development layer, and the reagent component is formed on the same plane as the development layer, thereby making it easy to manufacture the biosensor.

Further, the parallel side faces of the development layer are melted by a laser and cured so as to be sealed, whereby a required part of the parallel side faces can be melted and cured more speedily and uniformly and the melted and cured part can be given uniformity. Further, by the laser melting, the parallel side faces can be melted and cured in no physical contact with the development layer, so that the shape of the development layer is not changed due to contact pressure, resulting in a high-accuracy measurement.

### (Example)

Hereinafter, the present invention will be described more specifically through an example, while the present invention is not restricted to descriptions in this example in the scope of the invention.

In this embodiment, the immuno-chromatography development layer which includes an anti-hCG-β antibody immobilization line and a broad band of a complex of an anti-hCG-α antibody and gold colloid in a nitrocellulose film was manufactured as described below, and the liquid-impermeable sheet material was provided on the immuno-chromatography development layer and the base material was provided thereunder. Thereafter, an immuno-chromatography specimen which was cut employing a CO₂ laser and an immuno-chromatography specimen which was cut with a cutting tool (score cutter) were manufactured to form an individual biosensor, and a measurement of hCG in urine was performed employing respective immuno-chromatography specimens to compare variation of their measurement values.

### A. Preparation of immuno-chromatography development layer

First, the anti-hCG-β antibody solution which was diluted with a phosphate buffer solution to control the concentration was prepared. This antibody solution was applied on the nitrocellulose film by employing a solution discharge device. Thereby, a detecting antibody immobilization line was obtained on the nitrocellulose film. After being dried, this nitrocellulose film was immersed in a Tris-HCl buffer solution including a 1% skim milk, and gently shaken for 30 minutes. 30 minutes later, the film was moved into a Tris-HCl buffer solution tank, gently shaken for 10 minutes, and thereafter gently shaken in another Tris-HCl buffer solution tank for another 10 minutes, thereby washing the film. After washed twice as described above, the film was taken out from the cleaning fluid and dried at room temperature.

The gold colloid was prepared by adding 1% citric acid solution to a refluxing 100°C-solution of 0.01% chloroauric acid. After the reflux was continued for 30 minutes, the golod colloid was cooled and prepared to pH9 by using a 0.2M potassium carbonate solution. The anti-hCG-α antibody was added to this gold colloid solution, then the obtained solution was stirred for several minutes, and thereafter a 10% BSA (bovine serum albumin) solution pH9 was added thereto by such an amount that a 1% solution was finally obtained, and stirred. Thereby, an antibody-gold colloid complex (marked antibody) solution was prepared. Thereafter, this marked antibody solution was centrifuged at 4°C and 20000G for 50 minutes, whereby the marked antibody was isolated, and the isolated marked antibody was suspended in a cleaning buffer solution (1% BSA · phosphate buffer solution) and thereafter centrifuged to wash and isolate the marked antibody. After suspended in the cleaning buffer solution and filtrated through a 0.8µm filter, the marked antibody was prepared to be one-tenth as much as the initial gold colloid solution and stored at 4°C.

The so-created marked antibody solution was set in the solution discharge device and applied to a position apart from the antibody immobilization position on the anti-hCG-β antibody immobilization dry film, and thereafter the film was dried. Thereby, the marked antibody hold region was obtained on the immobilization film.

In this way, the immuno-chromatography development layer can be completed.

### B. Creation of immuno-chromatography specimen

The immuno-chromatography development layer manufactured as described above was attached onto the base material made of white PET with 0.5mm thickness, and the liquid-impermeable sheet made of transparent PET with 100µm thickness was attached onto the development layer from the marked antibody holding part to the end of the development layer with a part for laminating the water absorbing part opened at the end. Then, there were manufactured the immuno-chromatography specimen which was cut with the width of 2.5mm employing a CO₂ laser, in which the side faces of the development layer are melted and cured to be sealed, and the immuno-chromatography specimen which was cut with a cutting tool (score cutter). Further, glass fiber filter paper is attached respectively to the ends as the water absorbing parts. In this way, two kinds of immuno-chromatography specimens can be manufactured. While in the example the glass fiber filter paper is attached as the water absorbing part, the water absorbing part is not indispensable, and it is also possible that the immuno-chromatography development layer except for its beginning, where the inspection target solution is applied, and end is adherently covered with the liquid-impermeable sheet, thereby providing the end of the development layer with the same effect as that of the water absorbing part. That is, by opening the end of the development layer, the inspection target solution is easily evaporated and it is utilized that the level of the inspection target solution which reached to the end of the development layer comes level with that of the inspection target solution in the beginning in relation to the water head difference.

### C. Preparation of inspection target solution

The hCG solutions of known concentrations were added to human urine, thereby preparing the hCG solutions of various known concentrations.

### D. Measuring method of hCG in urine

More than 20µl of urine including hCG was applied to sample application parts on the two kinds of immuno-chromatography specimens created as described above and developed in the direction of the water absorbing parts, to cause an antigen antibody reaction, whereby color reactions in antibody immobilization parts were caused. Here, the coloration states 5 minutes after the sample was applied to the specimens were measured by employing a reflective spectrophotometer (CS9300; Shimadzu Corporation made), and the coloration degree was computed.

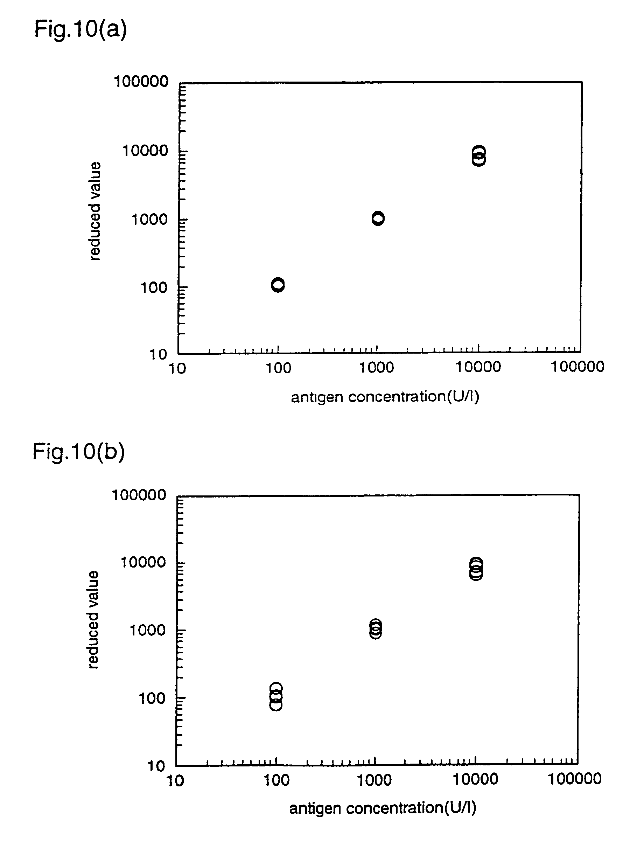

Figures 10 are graphs illustrating relationships between hCG concentration and a measurement result in the above-described two kinds of immuno-chromatographic specimens according the example of the present invention; figure 10(a) illustrates the measurement result in the immuno-chromatographic specimen cut employing a CO₂ laser and figure 10(b) illustrates the measurement result in the immuno-chromatographic specimen cut with a cutting tool (score cutter).

First, urine including each hCG of hCG concentration 100, 1000, and 10000U/l were applied to the immuno-chromatography specimen to be developed. Then, the coloration state of the antibody immobilization part on the specimen for urine of each hCG concentration was measured by the reflective spectrophotometer. In the example, an absorbance at the wavelength of 520nm was measured by the reflective spectrophotometer, and substituted into a previously formed calibration curve indicating a relationship between the hCG concentration and the absorbance, thereby obtaining a reduced value.

As a result, CV values (coefficients of variation) of the specimen cut employing a CO₂ laser shown in figure 10(a) were 3-10%, while the CV values of the specimen cut with a cutting tool (score cutter) shown in figure 10(b) varied widely between 20% and 35%. As described above, high quantitative accuracy was confirmed in the measurement employing the specimen cut employing a CO₂ laser.

### APPLICABILITY IN INDUSTRY

A biosensor and a manufacturing method thereof according to the present invention are quite useful as a biosensor which improves accuracy of a result of a reaction between a liquid sample as an analysis target and a marked reagent, and as a biosensor manufacturing method for simplifying a manufacturing process of such high-accuracy biosensor as well as reducing the cost.

## Claims

1. A biosensor which is provided with a development layer (2) for developing an inspection target solution, includes a region (5) of an immobilized reagent which is immobilized in a part of the development layer (2) that includes its side faces (8) parallel to the direction of the inspection target solution permeating and a region (4) of a marked reagent which is held in a part of the development layer (2) that includes its side faces (8) parallel to the direction of the inspection target solution permeating, in a marked dry state to be eluted by development of the inspection target solution, and measures a bonding amount of the marked reagent in the immobilized reagent region (5), thereby qualitatively or quantitatively measuring a measurement component in the inspection target solution, wherein
a reagent component on the side faces (8) of the development layer (2) parallel to the inspection target solution permeating, in the marked reagent region (4) and the immobilized reagent region (5) is denatured to be deactivated (9).

2. The biosensor as defined in claim 1, wherein
the side faces (8) of the development layer (2) parallel to the direction of the inspection target solution permeating are partially or entirely melted and cured to be sealed.

3. The biosensor as defined in claim 1 or claim 2, wherein
the surface or surface and rear face of the development layer except for a part for applying the inspection target solution is covered with a liquid-impermeable sheet (6), and
the side faces (8) of the development layer (2) and liquid-impermeable sheet parallel to the direction of the inspection target solution permeating and partially or entirely melted and cured to be sealed.

4. The biosensor as defined in claim 1 or claim 2, wherein
the surface or surface and rear face of the development layer except for parts at the beginning and end in the direction of the inspection target solution being applied is covered with a liquid-impermeable sheet (6), and
the side faces (8) of the development layer (2) and liquid-impermeable sheet (6) parallel to the direction of the inspection target solution permeating are partially or entirely melted and cured to be sealed.

5. The biosensor as defined in any one of claims 1 to 4, wherein
the development layer (2) is composed of a nitrocellulose.

6. The biosensor as defined in any one of claims 1 to 5, wherein
the parallel side faces (8) of the development layer (2) are melted by a laser and cured so as to be sealed.

7. The biosensor as defined in any one of claims 1 to 6, wherein
the whole sensor including the immobilized reagent and the marked reagent (4) is in a dry state.

8. The biosensor as defined in any one of claims 1 to 7, wherein
the biosensor is an immuno-chromatography specimen in which a complex of the immobilized reagent and the marked reagent is formed on a permeable porous material, thereby performing a measurement.

9. The biosensor as defined in any of claims 1 to 3, wherein
the biosensor is a one-step immuno-chromatography specimen in which a measurement is performed on a permeable porous material by an applying operation of the inspection target solution.

10. The biosensor as defined in any one of claims 1 to 9, wherein
the immobilized reagent and the marked reagent are formed on the same plane and member.

11. A method for manufacturing a biosensor which is provided with a development layer (2) for developing an inspection target solution, includes a region of an immobilized reagent (5) which is immobilized in a part of the development layer (2) that includes its side faces (8) parallel to the direction of the inspection target solution permeating and a region (4) of a marked reagent which is held in a part of the development layer (2) that includes its side faces (8) parallel to the direction of the inspection target solution permeating, in a marked dry state to be eluted by development of the inspection target solution, and measures a bonding amount of the marked reagent in the immobilized reagent region (5), thereby qualitatively or quantitatively measuring a measurement component in the inspection target solution, including
a denaturing and deactivating process for denaturing and deactivating (9) a reagent component on the side faces (8) parallel to the inspection target solution permeating, in the marked reagent region (4) and the immobilized reagent region (5).

12. The biosensor manufactunng method as defined in claim 11, wherein
the denaturing and deactivating process is implemented by irradiating a laser partially or entirely to the side faces (5).

13. A method according to claim 11, further including
a melting process for melting and curing side faces (8) of the development layer (2) which are parallel to the direction of the inspection target solution permeating to seal the same partially or entirely.

14. The biosensor manufacturing method as defined in claim 12, wherein
the melting process is implemented by irradiating a laser to the side faces (8) of the development layer (2) partially or entirely.

15. A method according to claim 11, further including
a cutting and melting process for cutting the sheet-shaped development layer (2) in parallel to the direction of the inspection target solution permeating, and simultaneously melting and curing cut surfaces (8) of the development layer (2) to seal.

16. The biosensor manufacturing method as defined in claim 15, wherein
the cutting and melting process is implemented by trimming the sheet-shaped development layer (2) by a laser.

## Patentansprüche

1. Biosensor mit einer Entwicklungsschicht (2) zum Entwickeln einer Untersuchungsziellosung, wobei der Biosensor einen Bereich umfasst mit einem immobilisierten Reagens (5), das in einem Teil der Entwicklungsschicht (2) immobilisiert ist, der ihre zu der Durchdringungsrichtung der Untersuchungsziellosung parallelen Seitenflachen (8) umfasst, und einen Bereich umfasst mit einem markierten Reagens (4), das in einem Teil der Entwicklungsschicht (2) gehalten wird, der ihre zu der Durchdringungsrichtung der Untersuchungsziellosung parallelen Seitenflachen (8) umfasst, in einem markierten trockenen Zustand, um durch die Entwicklung der Untersuchungsziellosung eluiert zu werden, und der einen Bindungsanteil des markierten Reagens in dem Bereich mit dem immobilisierten Reagens (5) misst, dabei qualitativ oder quantitativ eine Messkomponente in der Untersuchungsziellosung messend, wobei
eine Reagenskomponente auf den zur Durchdringungsrichtung der Untersuchungsziellosung parallelen Seitenflachen (8) der Entwicklungsschicht (2) in dem Bereich mit dem markierten Reagens (4) und dem Bereich mit dem immobilisierten Reagens (5) denaturiert wird, so dass sie deaktiviert wird (9)

2. Biosensor nach Anspruch 1, wobei
die zu der Durchdringungsrichtung der Untersuchungsziellosung parallelen Seitenflachen (8) der Entwicklungsschicht (2) teilweise oder vollig geschmolzen und ausgehartet sind, so dass sie versiegelt sind

3. Biosensor nach Anspruch 1 oder 2, wobei
die Oberflache oder die Oberflache und die Ruckseitenflache der Entwicklungsschicht mit Ausnahme eines Teiles zum Applizieren der Untersuchungsziellosung mit einer flussigkeitsundurchdringbaren Bedeckung (6) bedeckt sind und
die zu der Durchdringungsrichtung der Untersuchungsziellösung parallelen Seitenflachen (8) der Entwicklungsschicht (2) und die flüssigkeitsundurchdringbare Bedeckung (6) teilweise oder vollstandig geschmolzen und ausgehartet sind, so dass sie versiegelt sind

4. Biosensor nach Anspruch 1 oder 2, wobei
die Oberfläche oder Rückseitenflache der Entwicklungsschicht mit Ausnahme von Teilen am Anfang und am Ende in der Richtung, in der die Untersuchungsziellosung appliziert wird, bedeckt ist mit einer flussigkeitsundurchdringbaren Bedeckung (6) und
die zu der Durchdringungsrichtung der Untersuchungsziellosung parallelen Seitenflachen (8) der Entwicklungsschicht (2) und die flussigkeitsundurchdringbare Bedeckung (6) teilweise oder vollstandig geschmolzen und ausgehartet sind, so dass sie versiegelt sind.

5. Biosensor nach einem der Anspruche 1 bis 4, wobei
die Entwicklungsschicht (2) aus einer Nitrozellulose gebildet wird.

6. Biosensor nach einem der Anspruche 1 bis 5, wobei
die parallelen Seitenflachen (8) der Entwicklungsschicht (2) mittels eines Lasers geschmolzen werden und ausgehartet werden, so dass sie versiegelt sind.

7. Biosensor nach einem der Anspruche 1 bis 6, wobei
der gesamte Sensor einschließlich des immobilisierten Reagens und des markierten Reagens (4) in einem trockenen Zustand ist.

8. Biosensor nach einem der Anspruche 1 bis 7, wobei
der Biosensor eine Immuno-Chromatographieprobe ist, in der ein Komplex des immobilisierten Reagens und des markierten Reagens gebildet wird auf einem durchdringbaren porosen Material, wodurch eine Messung durchgeführt wird.

9. Biosensor nach einem der Anspruche 1 bis 3, wobei
der Biosensor eine Einschritt-Immuno-Chromatographieprobe ist, in der eine Messung auf einem durchdringbaren porosen Material durchgeführt wird mittels eines Applizierungsvorganges der Untersuchungsziellosung.

10. Biosensor nach einem der Anspruche 1 bis 9, wobei
das immobilisierte Reagens und das markierte Reagens auf derselben Ebene und demselben Teil gebildet werden.

11. Verfahren zum Herstellen eines Biosensors, der versehen ist mit einer Entwicklungsschicht (2) zum Entwickeln einer Untersuchungsziellösung, der einen Bereich umfasst mit einem immobilisierten Reagens (5), das in einem Teil der Entwicklungsschicht (2) immobilisiert ist, der ihre zu der Durchdringungsrichtung der Untersuchungsziellosung parallelen Seitenflächen (8) umfasst, und einen Bereich umfasst mit einem markierten Reagens (4), das in einem Teil der Entwicklungsschicht (2) gehalten wird, der ihre zu der Durchdringungsnchtung der Untersuchungsziellosung parallelen Seitenflachen (8) umfasst, in einem markierten trockenen Zustand, um durch die Entwicklung der Untersuchungszrellosung eluiert zu werden, und der einen Bindungsanteil des markierten Reagens in dem Bereich fur das immobilisierte Reagens (5) misst, dabei qualitativ oder quantitativ eine Messkomponente in der Untersuchungsziellosung messend, das einschließt ein Denaturierungs- und Deaktivierungsverfahren zum Denaturieren und Deaktivieren (9) einer Reagenskomponente auf den zur Durchdringungsrichtung der Untersuchungsziellosung parallelen Seitenflachen (8) in dem Bereich mit dem markierten Reagens (4) und dem Bereich mit dem immobilisierten Reagens (5).

12. Verfahren zum Herstellen eines Biosensors nach Anspruch 11, wobei
das Denatunerungs- und Deaktivierungsverfahren durchgeführt wird durch teilweises oder vollstandiges Bestrahlen der Seitenflachen (5) mit einem Laser.

13. Verfahren nach Anspruch 11, das weiter umfasst
ein Schmelzverfahren zum Schmelzen und Ausharten der zu der Durchdringungsrichtung der Untersuchungsziellosung parallelen Seitenflachen (8) der Entwicklungsschicht (2), um diese teilweise oder vollstandig zu versiegeln.

14. Verfahren zum Herstellen eines Biosensors nach Anspruch 12, wobei
das Schmelzverfahren durchgeführt wird durch teilweises oder volistandiges Bestrahlen der Seitenflachen (8) der Entwicklungsschicht (2) mit einem Laser.

15. Verfahren nach Anspruch 11, das weiter umfasst
ein Schneide- und Schmelzverfahren zum Schneiden der blattformigen Entwicklungsschicht (2) parallel zu der Durchdringungsrichtung der Untersuchungsziellosung und gleichzeitiges Schmelzen und Ausharten der Schnittflächen (8) der Entwicklungsschicht (2) zum Versiegeln.

16. Verfahren zum Herstellen eines Biosensors nach Anspruch 15, wobei
das Schneide- und Schmelzverfahren durchgeführt wird durch Schneiden der blattformigen Entwicklungsschicht (2) mittels eines Lasers.

## Revendications

1. Biocapteur qui est fourni avec une couche (2) de développement destinée à développer une solution cible d'inspection, comportant une région (5) d'un réactif immobilisé qui est immobilisé dans une partie de la couche (2) de développement qui comprend ses faces latérales (8) parallèles à la direction de diffusion de la solution cible d'inspection et une région (4) d'un réactif marqué qui est maintenu dans une partie de la couche (2) de développement qui comprend ses faces latérales (8) parallèles à la direction de la diffusion de la solution cible d'inspection, dans un état sec marqué élué par le développement de la solution cible d'inspection, et mesure une quantité de liaison du réactif marqué dans la région (5) du réactif immobilisé, mesurant ainsi qualitativement ou quantitativement un composant de mesure dans la solution cible d'inspection, dans lequel
un composant de réactif sur les faces latérales (8) de la couche (2) de développement parallèle à la direction de la diffusion de la solution cible d'inspection, dans la région (4) du réactif marqué et la région (5) du réactif immobilisé est dénaturé pour être désactivé (9).

2. Biocapteur selon la revendication 1, dans lequel
les faces latérales (8) de la couche (2) de développement parallèles à la direction de la diffusion de la solution cible d'inspection sont partiellement ou entièrement fondues et durcies pour être scellées.

3. Biocapteur selon la revendication 1 ou la revendication 2, dans lequel
la surface ou la surface et la face arrière de la couche de développement, sauf une partie destinée à appliquer la solution cible d'inspection, est recouverte d'une feuille imperméable au liquide (6), et
les faces latérales (8) de la couche (2) de développement et la feuille imperméable au liquide parallèles à la direction de la diffusion de la solution cible d'inspection sont partiellement ou entièrement fondues et durcies pour être scellées.

4. Biocapteur selon la revendication 1 ou la revendication 2, dans lequel
la surface ou la surface et la face arrière de la couche de développement, sauf des parties au début et à la fin dans la direction de la solution cible d'inspection en train d'être appliquée, est recouverte d'une feuille imperméable au liquide (6), et
les faces latérales (8) de la couche (2) de développement et la feuille imperméable au liquide (6) parallèles à la direction de la diffusion de la solution cible d'inspection sont partiellement ou entièrement fondues et durcies pour être scellées.

5. Biocapteur selon l'une quelconque des revendications 1 à 4, dans lequel
la couche (2) de développement est composée d'une nitrocellulose.

6. Biocapteur selon l'une quelconque des revendications 1 à 5, dans lequel
les faces latérales parallèles(8) de la couche (2) de développement sont fondues par un laser et durcies de façon à être scellées.

7. Biocapteur selon l'une quelconque des revendications 1 à 6, dans lequel
tout le capteur comprenant le réactif immobilisé et le réactif marqué (4) est dans un état sec.

8. Biocapteur selon l'une quelconque des revendications 1 à 7, dans lequel
le biocapteur est un spécimen d'immuno-chromatographie dans lequel un complexe du réactif immobilisé et le réactif marqué est formé sur un matériau poreux perméable, réalisant ainsi une mesure.

9. Biocapteur selon l'une quelconque des revendications 1 à 3, dans lequel
le biocapteur est un spécimen d'immuno-chromatographie à une étape dans lequel on réalise une mesure sur un matériau poreux perméable par une opération de mise en oeuvre de la solution cible d'inspection.

10. Biocapteur selon l'une quelconque des revendications 1 à 9, dans lequel
le réactif immobilisé et le réactif marqué sont formés sur le même plan et le même élément.

11. Procédé de fabrication d'un biocapteur qui est fourni avec une couche (2) de développement destinée à développer une solution cible d'inspection, comprenant une région d'un réactif immobilisé (5) qui est immobilisé dans une partie de la couche (2) de développement qui comprend ses faces latérales (8) parallèles à la direction de la diffusion de la solution cible d'inspection et une région (4) d'un réactif marqué qui est maintenu dans une partie de la couche (2) de développement qui comprend ses faces latérales (8) parallèles à la direction de la diffusion de la solution cible d'inspection, dans un état sec marqué à éluer par le développement de la solution cible d'inspection, et mesure une quantité de liaison du réactif marqué dans la région (5) du réactif immobilisé, mesurant ainsi qualitativement ou quantitativement un élément de mesure dans la solution cible d'inspection, comprenant un procédé dénaturant et désactivant destiné à dénaturer et à désactiver (9) un composant de réactif sur les faces latérales (8) parallèles à la diffusion de la solution cible d'inspection, dans la région (4) du réactif marqué et dans la région (5) du réactif immobilisé.

12. Procédé de fabrication du biocapteur selon la revendication 11, dans lequel le processus de dénaturation et de désactivation est mis en oeuvre en envoyant des radiations à l'aide d'un laser partiellement ou entièrement sur les faces latérales (5).

13. Procédé selon la revendication 11, comprenant en outre
un processus de fusion pour fondre et durcir des faces latérales (8) de la couche (2) de développement qui sont parallèles à la direction de la diffusion de la solution cible d'inspection pour sceller ces dernières partiellement ou entièrement.

14. Procédé de fabrication du biocapteur selon la revendication 12, dans lequel
le processus de fusion est mis en oeuvre en envoyant des radiations à l'aide d'un laser sur les faces latérales (8) de la couche (12) de développement partiellement ou entièrement.

15. Procédé selon la revendication 11, comprenant en outre
Un processus de découpe et de fusion destiné à découper la couche (2) de développement en forme de feuille parallèlement à la direction de la diffusion de la solution cible d'inspection, et en fondant et durcissant simultanément des surfaces (8) coupées de la couche (2) de développement à sceller.

16. Procédé de fabrication du biocapteur selon la revendication 15, dans lequel le processus de découpe et de fusion est mis en oeuvre en ébavurant la couche (2) de développement en forme de feuille à l'aide d'un laser.
